# EUROPEAN PATENT APPLICATION

(11) **EP 1 018 327 A1**
(43) Date of publication of application: **12.07.2000**
(21) Application number: 99100119.9
(22) Date of filing: 04.01.1999
(51) Int. Cl.: A61F 5/451

(54) **Container for the collection of bodily waste with an anatomically shaped flange**

(71) Applicant: THE PROCTER & GAMBLE COMPANY, Cincinnati, Ohio 45202 (US)
(72) Inventor: d'Acchioli, Vincenzo, 65779 Kelkheim am Taunus (DE); Palumbo, Gianfranco, 61348 Bad Homburg (DE); Evangelista, Olindo, 66023 Francavilla al Mare, Chieti (IT); Gonzalez, Denis Alfred, 65129 Pescara (IT)
(74) Representative: Kohol, Sonia

(57) **Abstract**

The present invention relates human waste management device, such as urine management devices and faecal management devices, these devices comprising a bag for storage of human waste and being suitable for babies, children or adults. The invention resides principally in providing such devices with an anatomically shaped flange.

## Description

### Field of the invention

The present invention relates to urine management devices and to faecal management devices, all comprising storage means and all being suitable for babies, children or adults. Said devices are provided with a anatomically shaped flange.

### Background of the invention

A problem naturally associated with these devices is their attachment to the human body. The approach which is mostly used in the field is to provide the device with an adhesive flange, which will stick to the perianal or genital area.

However, reliable adhesive attachment is not readily achieved. Even unintentional detachment is known in the art, as mentioned in GB 2 116 849. Such unintentional detachment will typically be associated with leakage and thus lead to most unwanted consequences. The problem occurs to some extent because the adhesive should be chosen not to be too aggressive in order to allow convenient detachment of the device and avoidance of negative effects on the wearer's skin. Many wearers, who make use of faecal or urine management devices have sensitive skin due to their age, whether very old or very young, and furthermore sometimes also suffer from skin irritations. Proper fit of the device, namely the flange, supports the wearing comfort, the skin friendliness and the reliable adhesion of the device.

Urine management devices are, for instance, disclosed in the following documents: GB 1,092,274 discloses a pediatric urine collector for female use comprising a collector bag of plastic material opening. The base of the opening is provided with a wedge like projection adapted to engage the lower perineal area of the infant. The collector is secured to the body of the wear by adhesive material. US 4,804,377 discloses a collector for urine specimens from children. The collector comprises a rectangular flange for adhesive attachment, which comprises a round, slightly oval aperture. US 1,092,274 discloses a urine collector for female infants comprising an aperture which is smaller at the bottom end than at the top end and could be described as droplet shaped. US 3,292,626 also discloses a urine collector for female infants comprising a circular or droplet shaped aperture. Chinese patent application CN 1079381 discloses urine bags for infants with circular and elliptical apertures.

In dealing with the problems outlined above concerning reliable adhesive attachment US 3,522,807 and US 3,734,096, for example, disclose faecal receptacles having an adhesive flange surrounding the aperture in the device, for attachment to the body of the patient in nursing or medical applications; the flange contains a plurality of tabs extending outwardly from the aperture and said tabs are covered with adhesive in the same manner as the rest of the flange and thus are designed to serve as adhering aids.

US 3,522,807 and US 3,734,096 further describe ing-like flanges, which correspond to the circular form of the sphincter muscle. US 5,593,397 discloses an approach of how to better adapt a ring-like flange surrounding a circular aperture to the anatomy of the wearer. A triangular portion of the flange is marked and those markings are intended to guide the caretaker in culling out a triangle of the flange, e.g. by using scissors. Such an adaptation of the flange is thought to improve the fit of the faecal management device in the perineal area of the wearer.

Similarly, US 4,784,656 discloses a device provided with a small circular aperture, the flange of which is provided with markings to allow to cut out circular apertures with different diameters. This may be beneficial to better adapt the faecal management device to the anatomy of different wearers, namely the size of the anus, however the use a cutting device, such as scissors, has several drawbacks. The required cutting device may not be at hand, the cutting device may cause injuries to a person or damage to the faecal management device, the cutting is a time consuming process and furthermore may not be accurate.

Another solution to this problem is brought up in GB-A-2,116,849. Again, a flange having a circular outer contour is used in combination with a circular aperture. Here, the aperture and the flange are not concentric, so that the flange has a smaller width at one end and may better fit the perineal area of the wearer.

In US 3,577,989 a device is disclosed, which has a non circular, in fact very long flange. This device, however, is meant to entrap both, urine and faeces, and is as such a device of a different kind. The aperture thus being long enough to cover the urinary duct and the anal opening. The patent further discloses the attachment of the device by the use of elastic straps. For most applications, however, adhesive attachment is prefered.

Patent applications PCT/US98/13412 and EP 97110604.2 disclose a flange for a faecal management device which is essentially flat, which however, comprises front and/or rear projections. These projections are designed to fit the perineal and the coccygeal area of a wearer in order to ensure a better sealing against leakage, while the flange as such is flat.

Besides and in connection with optimum adherence, the correct placement of the device is also a key issue in the field of human waste management devices, particularly faecal management devices. Total or substantial misplacement of the device will lead to a severe misfunctioning, in particular incomplete collection of faeces and leakage. If the aperture of the faecal management device is not sufficiently in registry with the anal opening, substantial pressure, in particular on the flanges of the device, can build up in the defecation process. Such substantial pressure can lead to the detachment of the adhesively secured device.

If the misplacement of the device is recognised before use, the placement of the device is normally corrected, typically by the carer. The necessary detachment and reattachment of the device causes an additional stress on the affected areas of skin of the wearer. Proper placement of the device in the first place is therefore highly desirable.

Hence, there still exists a need for human waste management devices and in particular for faecal management devices which can be easily and correctly positioned onto the desired area of the wearer by the caretaker or wearer themselves without causing discomfort to the wearer. Furthermore, there is a need for a device which optimal anatomical fit and reliable adhesive attachment.

It has now been found, that all of the above outlined problems can be addressed by providing a human waste management device with an anatomically adapted flange, which typically provides a three dimensional configuration.

### Summary of the invention

The present invention relates to human waste management device suitable for use for babies, children or adults. The invention resides principally in providing such devices with an anatomically adapted flange, which typically provides a threedimensional contour.

### Brief description of the drawings

It is believed that the invention will be better understood from the foregoing description in conjunction with the accompanying drawings in which:
Figure 1 is a perspective view of a preferred faecal management device according to the present invention.
Figure 2 is a perspective view of a faecal management device according to the prior art.
Figure 3 is a side view from one side of the line L of Figure 1.
Figure 4 is a cross sectional view along line T of Figure 1.
Figure 5 is a cross schematic view of parts of the human body including the perianal area. l₁ is a line following the contour of the body in the longitudinal direction, l₂ is a line following the contour of the body in the transversal direction.
Figure 6 is a side view (from one side of the longitudinal axis) of another preferred embodiment of the present invention.
Figure 7 is a side view (from one side of the longitudinal axis) of another preferred embodiment of the present invention.
Figure 8 is a transversal cross-section of another preferred embodiment of the present invention.
Figure 9 is a side view (from one side of the longitudinal axis) of another preferred embodiment of the present invention.
Figure 10 is a side view (from one side of the longitudinal axis) of a prior art embodiment.

### Detailed description of the invention

The invention relates to human waste management devices. Such a human waste management device may be a faecal management device (10), which is designed for attachment to the anal area and mainly used for collecting faeces, or it may be a urine management device, which is attached to the urinary duct and mainly used for collecting urine. A human waste management device may also be a device to collect both urine and faeces and thus be attached to both of the above areas. All of the above human waste management devices are preferably designed for single use and disposal thereafter.

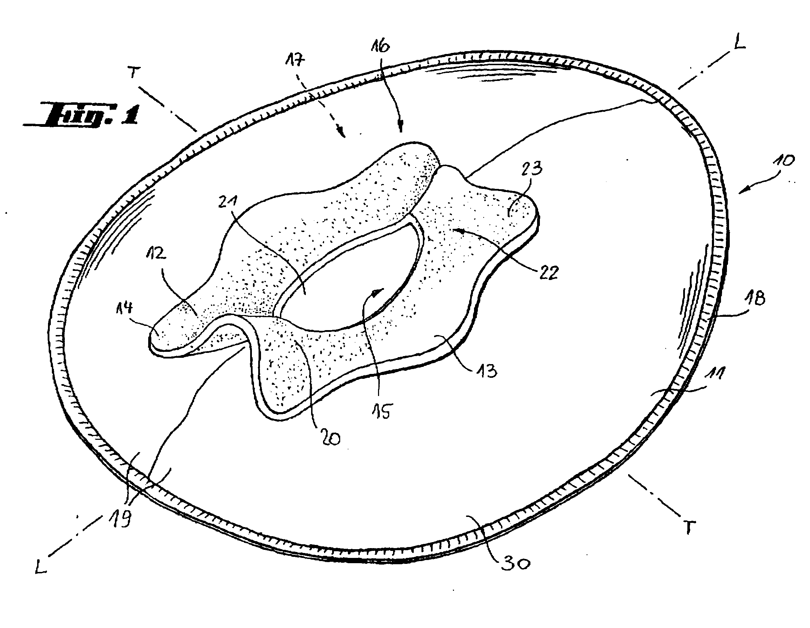

A faecal management device (10) is shown in Figure 1.

### Description of the human waste management device as a whole

Typically human waste management devices comprise a bag (11) having an aperture (21) and a flange (12) surrounding the aperture for preferably adhesive attachment to the perianal area of a wearer as visible from Figure 1. Any human waste management device known in the art can be provided according to the present invention.

The bag (11) as used herein is a flexible receptacle for the containment of excreted faecal matter or urine. The bag (11) is designed to safely contain any entrapped material, typically it will be liquid impermeable, yet it may be breathable. The bag (11) is designed of sufficient strength to withstand rupture in use, also when pressure on the bag (11) is exerted in typical wearing conditions, such as sitting.

According to the present invention the bag material can comprise one or multiple layers, preferably two or three layers. The layer on the inside of the bag (11), which will typically at least partially come in contact with faecal material or urine is called the inner layer. The outermost layer of the bag, which will typically at least partially come in contact with the skin to the wearer and the garments of the wearer, is called the outer layer.

The layers of the bag material may comprise any material, preferably so that the bag is liquid impervious. The preferred optical properties of any such material will be described below in more detail. The layers may in particular comprise any material such as non-wovens or films. In a preferred embodiment of the present invention a laminate may be formed from a non-woven layer and a film. The laminate can be formed by means known to the man skilled in the art.

Any non-woven layer can comprise felt fabrics, spunlaced fabrics, fluid jet entangled fabrics, air-laid fabrics, wet-laid fabrics, dry-laid fabrics, melt-blown fabrics, staple fibre carding fabrics, spunbonded fabrics, stitch-bonded fabrics, apertured fabrics, combinations of the above or the like.

Suitable film materials for any of said layers preferably comprise a thermoplastic material. The thermoplastic material can be selected from among all types of hot-melt adhesives, polyolefins especially polyethylene, polypropylene, amorphous polyolefins, and the like; material containing meltable components comprising fibres or polymeric binders including natural fibres such as cellulose - wood pulp, cotton, jute, hemp; synthetic fibres such as fibreglass, rayon, polyester, polyolefin, acrylic, polyamid, aramid, polytetrafluroethylene metal, polyimide; binders such as bicomponent high melt/low melt polymer, copolymer polyester, polyvinyl chloride, polyvinyl acetate/chloride copolymer, copolymer polyamide, materials comprising blends wherein some of the constituent materials are not meltable; air and vapour permeable materials including microporous films such as those supplied by EXXON Chemical Co., Ill, US under the designation EXXAIRE or those supplied by Mitsui Toatsu Co., Japan under the designation ESPOIR NO; and monolithic breathable materials such as Hytrel™ available from DuPont and Pebax™ available from ELF Atochem, France.

In a preferred embodiment a film, which is comprised in any layer, is preferably permeable to gases such as air and to vapour such as water vapour in order to avoid the problem of entrapment and condensation of moisture vapour given off by the body of the wearer and thus, the hot, clammy and uncomfortable conditions after a short period of use.

The outer layer of the bag material may comprise a non-woven layer. Such material layers present an uneven surface to the skin of the wearer and thus reduce significantly the problem of occlusion and greatly improve skin healthiness.

In one preferred embodiment of the present invention the bag material comprises two layers. Preferably the outer layer comprises a non-woven layer and the inner layer comprises a film.

In yet another preferred embodiment of the present invention, the bag material comprises three layers, preferably one film and two non-woven layers. In an even more preferable embodiment the film is interposed between the two non-woven layers. This sequence of layers results in a closed fibrous structure, which has a particularly pleasing sensation on contact with the skin of the wearer. In yet another preferred embodiment the inner layer comprises a film and the other two layers comprise non-wovens.

The non-woven layer or the non-woven layers comprised by the bag material may be hydrophobic or hydrophilic. If the bag material does not comprise a film layer, preferably at least one non-woven layer is hydrophobic. As a consequence, fluid penetration is resisted through the wearer facing portion (16) and the garment facing portion (17) of the human waste management device (10). If the bag material comprises a film or a hydrophobic non-woven layer, further non-woven layers may be hydrophilic.

Typically, the non-woven layer is treated with a surface active material, such as a fluorchemical or other hydrophobic finishings, to provide the requisite hydrophobicity. The non-woven layer, however, may equally be treated with coatings of liquid impervious materials such as hot-melt adhesives or coatings of silicone or other hydrophobic compounds such as rubbers and vegetable and mineral waxes or it may be physically treated using nano-particulates or plasma coating techniques, for example.

The non-woven layer can also be treated with agents to improve the tactile perceivable softness of the wearer facing portion (16) and the garment facing portion (17). The agents include but are not limited to vegetable, animal or synthetic oils, silicone oils and the like. The presence of these agents are known to impart a silky or flannel-like feel to the non-woven layer without rendering it greasy or oily to the tactile sense of the wearer. Additionally, surfactant material, including anionic, non-anionic, cationic and non-cationic surfactants, may be added to further enhance softness and surface smoothness.

Furthermore, the non-woven layer may be impregnated with a lotion to provide desirable therapeutic or protective coating lotion benefits. The lotion coating on the wearer facing portion (16) and the garment facing portion (17) is transferable to the skin of the wearer by normal contact and wearer motion and/or body heat. Generally, mineral oil in the form of a lotion is recognised as being effective in imparting a soothing, protective coating to the skin of the wearer. It is also possible to impregnate the non-woven layer with a solid oil phase of cream formulation or to incorporate into the non-woven layer an array of pressure- or thermal- or hydrorupturable capsules containing for example, baby oil.

According to the present invention, depending on the shape of the bag (11) required, the bag (11) may be provided from a unitary piece of material or a number of separate pieces of material, which may be identical or different and which are sealed at their respective peripheries. The preferred shape of the bag depends in particular on the intended use thereof, i.e. whether the device is intended for bedridden patients or active patients suffering from incontinence or requiring an artificial bowel or for infants.

The bags described herein preferably have a wearer facing portion (16) and a garment facing portion (17), which both comprise separate pieces of material. The wearer facing portion (16) and the garment facing portion (17) are sealed at the periphery of the bag (11), thus creating a bag peripheral rim (18). The wearer facing portion (16) and the garment facing portion (17) may each independently comprise more than one section of material. Preferably the garment facing portion (17) comprises only one section of material; most preferably also the wearer facing portion (16) comprises only one section of material.

The wearer facing portion (16), the garment facing portion (17) and the pieces of material comprised by either of these portions are secured to each other by means known to the man skilled in the art, such as adhesive, thermobonding or pressure bonding in order to provide the desired bag configuration. The rim (18), at which the wearer facing portion (16) and the garment facing portion (17) are sealed together, may be provided inside the bag (11) rather than outside the bag (11), thus being coextensive with the inner surface (15) of the bag (11) rather than with the outer surface (30) of the bag (11).

Hence a variety of shapes of the bag is within the scope of the present invention. Particularly, preferred shapes are flat circular type bags, cone shaped bags, truncated cone shaped bags and pyramidal or truncated pyramidal shaped bags and flat T shaped bags.

In one embodiment of the present invention the bag (11) may contain absorbent material. The absorbent material may comprise any absorbent material which is capable of absorbing and retaining liquids. The absorbent material may comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

The absorbent material may be positioned in the bag (11) in any suitable manner. For example, the absorbent material may be loosely arranged within the bag or may be secured to the inner surface (15) of the bag (11). Any known techniques for securing absorbent material to nonwoven and film substrates may be used to secure the absorbent material to the inner surface (15) of the bag. The absorbent material may also be arranged to have any desired shape or configuration (e.g., rectangular, oval, circular, etc.).

The bag (11) is provided with an aperture (21) whereby faecal matter or urine is received from the body prior to storage within the bag cavity. The aperture (21) is surrounded by a flange (12) and may be provided in any shape or size, such as circular, oblong, heart shaped and may be symmetrical or asymmetrical, preferably the aperture has an oblong configuration either in the longitudinal or in the transversal direction or in both directions, e.g. the contours of the aperture are in the shape of two ellipses with the respective main axes being substantially perpendicular.

The flange (12) is attached to the bag (11) according to any means known to the man skilled in the art which may provide permanent or releasable attachment. Preferably however, the flange is attached to the bag by adhesive. Typically, the bag will be attached to the flange, towards the outer periphery of flange so as not to cause any obstruction for the entering faecal matter or urine.

The flange may be provided in any size depending on the wearer group for which the device is intended. Similarly the flange may be provided in any shape and preferably has a symmetrical shape preferably comprising a plurality of lobes (13).

The flange comprises a garment facing portion (22) and a wearer facing portion (23). In an preferred embodiment these are two large, substantially flat surfaces, however, the flange (12) may also comprise projections, a front projection (28) and/or a rear projection (29), in case of a faecal management device (10) designed to fit the perineal and/or coccygeal area of the wearer and in case of a urine management device (10) designed to fit the genital and/or perineal area.

The flange (12) should be made of soft, flexible and malleable material to allow easy placement of the flange (12) to the perianal or uro-genital area. Typical materials include nonwoven materials, wovens, open celled thermoplastic foams, closed-cell thermoplastic foams, composites of open celled foams and stretch nonwoven, and films. A closed-cell foam of polyethylene has been found effective, but more preferably an open celled polyurethane foam is used. Preferably, such foams have a thickness within the general range of 0.1 to 5 millimetres and a density of 5 to 250 g/m², more preferably 50 g/m². Other thermoplastic foam materials, or other suitable plastics sheet materials having the described properties of such foams (i.e., softness, pliability, stretchability, and contractability) might also be used. Preferably, the material of garment facing portion (22) of the flange (12) may extend into the defined aperture area so as to form a skirt or flap of material which prevents unintentional adhesion of the surface edges of the flange (12) defining the aperture (21) to one another during use.

According to the present invention the human waste management device (10) further comprises an attachment means to secure the device to the wearer. Such means include straps and more preferably comprises a body-compatible pressure sensitive adhesive (20) applied to the wearer facing portion (23) of the flange (12).

The adhesive (20) is preferably covered with a release means (not shown) in order to protect the adhesive (20), such as siliconized paper. The adhesive (20) can cover the entire wearer facing portion (23) of the flange (12), more preferably the flange (12) has at least one, preferably two to six non-adhesive portions. These portions may be adhesive free or may contain inactivated or covered adhesives. As is evident from Figure 1, the adhesive is in one preferred embodiment not applied to the entire wearer facing portion (23) of the flange (12), so as to provide lobes (13) on either side of the flange (12) which are non-adhesive and can thereby serve to facilitate placement and removal of the device whilst avoiding contact with the adhesive. These lobes (13) are however preferably also covered by the release means. Before application of the human waste management device (10) to the skin of the wearer, the release means if present is removed.

According to the present invention any medically approved water resistant pressure sensitive adhesive may be used to attach the device to the perianal or uro-genital area of the wearer, such as hydrocolloid adhesives and hydrogel adhesives. Particularly effective adhesives in providing the desired adhesive properties to secure the flange to the skin of the wearer at the sensitive perianal area, whilst allowing for relatively painless application and removal, are formed from crosslinking polymers with a plastisicer to form a 3-dimensional matrix.

The adhesive (20) can be applied to the wearer facing portion (23) of the flange (12) by any means known in the art such as slot coating, spiral, or bead application or printing. Typically the adhesive (20) is applied at a basis weight of from 20g/m² to 2500g/m², more preferably from 500g/m² to 2000g/m² most preferably from 700g/m² to 1500g/m² depending on the end use envisioned. For example, for human waste management devices (10) to be used for babies the amount of adhesive (20) may be less than for human waste management devices (10) designed for active adult incontinence sufferers.

The human waste management device (10) of the present invention has been found to be particularly useful and beneficial when used in conjunction with a garment, or diaper (50), preferably a disposable diaper.

### Detailed description of the flange

To allow a more detailed and clear description of the device (10), in the following paragraphs firstly a few terms will be defined, as used herein.

Regarding in particular the flange (12) the longitudinal axis is to be understood as follows: The direction which is substantially defined by a line connecting the coccyx, the perineum and the genital area for a wearer with the urine management device (10) in the intended wearing position defines the longitudinal direction. The longitudinal axis is an axis in the longitudinal direction, which crosses the centre of the aperture (21). The longitudinal axis is typically also an axis of symmetry.

The transversal axis is an axis perpendicular to said longitudinal axis, which crosses the centre of the aperture (21). The transversal direction, which is defined by the transversal axis, is generally aligned with the wearers hips.

The z-axis is an axis perpendicular to both the longitudinal and the transversal axes crossing the centre C of the aperture (21). The z-axis will typically point towards a wearers anus and is typically an axis of symmetry for the flange (12).

A cross sectional view, as used herein, is obtained by a cut along a plane comprising the z-axis. For a longitudinal cross sectional view the cutting plane further comprises the longitudinal axis. For a transversal cross sectional view the cutting plane further comprises the transversal axis.

The aperture (21) of the human waste management device (10) as used herein is to be understood as the part of the device (10) which receives faecal matter, which is then entrapped in the bag. The aperture (21) does not need to be open, when not receiving faecal matter. For example, the aperture (21) may be closed by a given mechanism, in particular after detachment.

The contour of the aperture (21) is defined by the inner periphery (25) of the flange (12). If the aperture (21) is provided with a skirt, this skirt does not define the contour of the aperture (21).

Centre of the aperture (21) as used herein is identical to the centre point C of the line along the longitudinal axis which connects the two points of the inner periphery of the flange (12) along the longitudinal axis.

According to the present invention a human waste management device, such as a faecal management device (10) or a urine management device, is provided with an anatomically shaped flange. All references to urine management devices as made herein refer to urine management devices which comprise a storage means such as a bag, which may also comprise absorbent material. Urine management devices which do not have any storage capacity and typically serve to close the urethra are not within the scope of the present invention.

While the present invention comprises urine management devices as well as faecal management devices the following description focuses on faecal management devices. However, the person skilled in the art has no difficulties to adapt a human waste management device to the contours of the genitals rather than to the contours of the anus following the teaching of the present invention.

The relaxed configuration of the flange (12), which is of interest in the context of the present application, is to be understood as a form of the flange when no outer forces are present other than gravity and similarly almost unavoidable forces. This form is typically visible when the device is evenly supported, for example lies on a table.

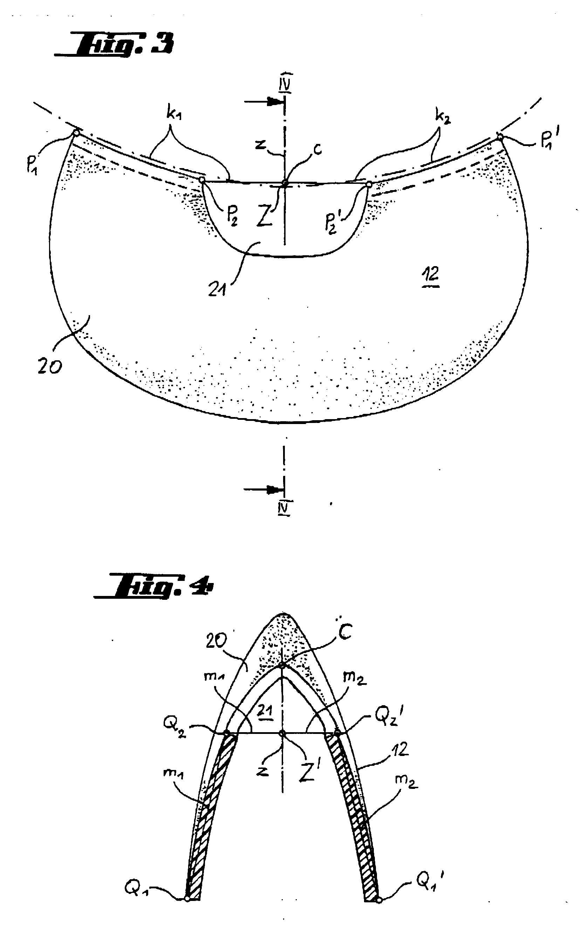

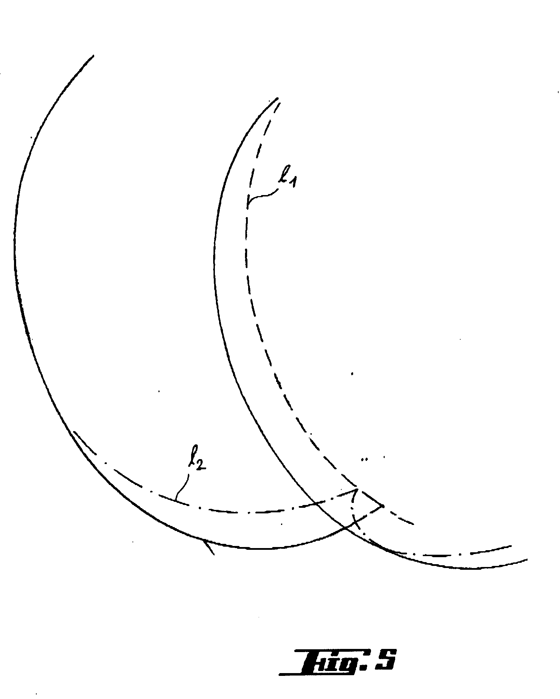

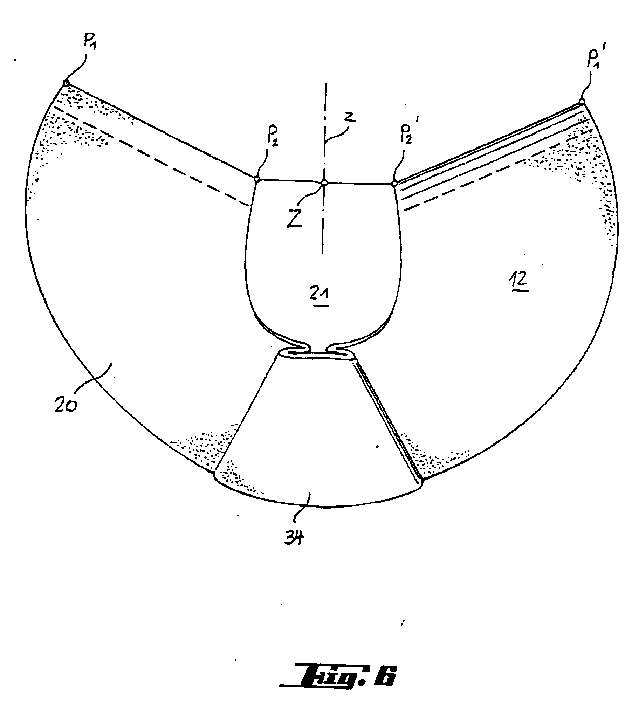

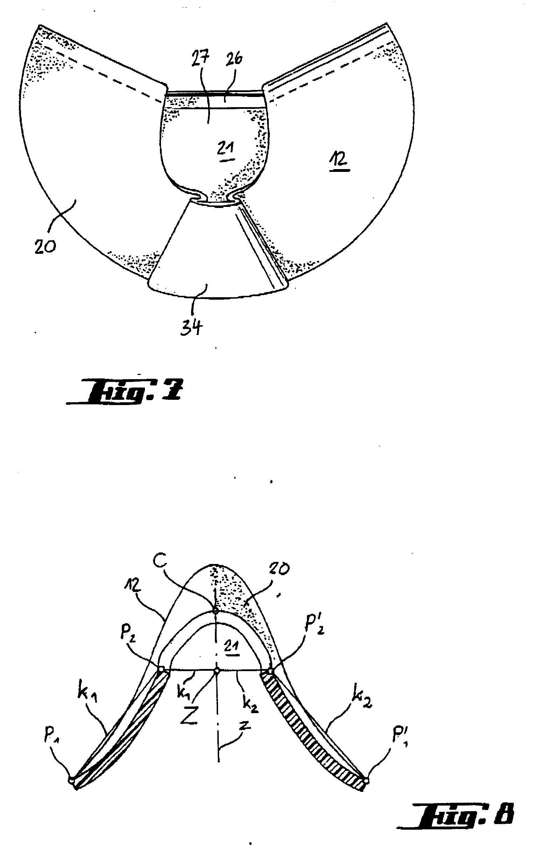

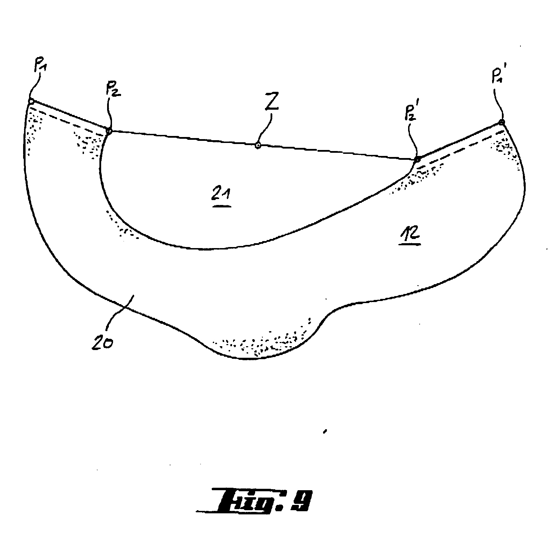

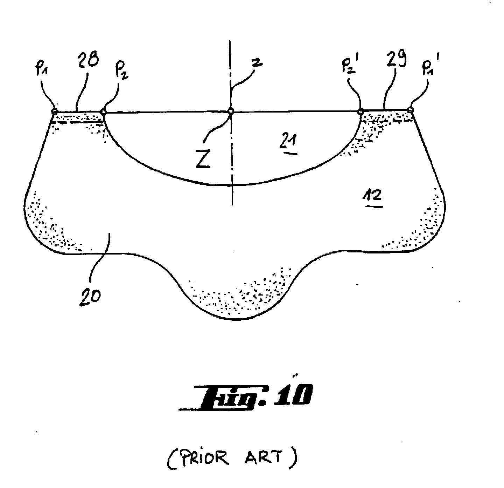

Figures 1, 3 and 4 show one preferred embodiment of the present invention. Referring to Figures 3 and 4 the flange (12) is adapted to follow the contours of the human body as schematically depicted in Figure 5. Namely, the curvature of the flange (12) as depicted in Figure 3, is designed to follow the contours of the human body along line l₁ of Figure 5, a line which extends from the coccyx via the anal opening to the perineum.

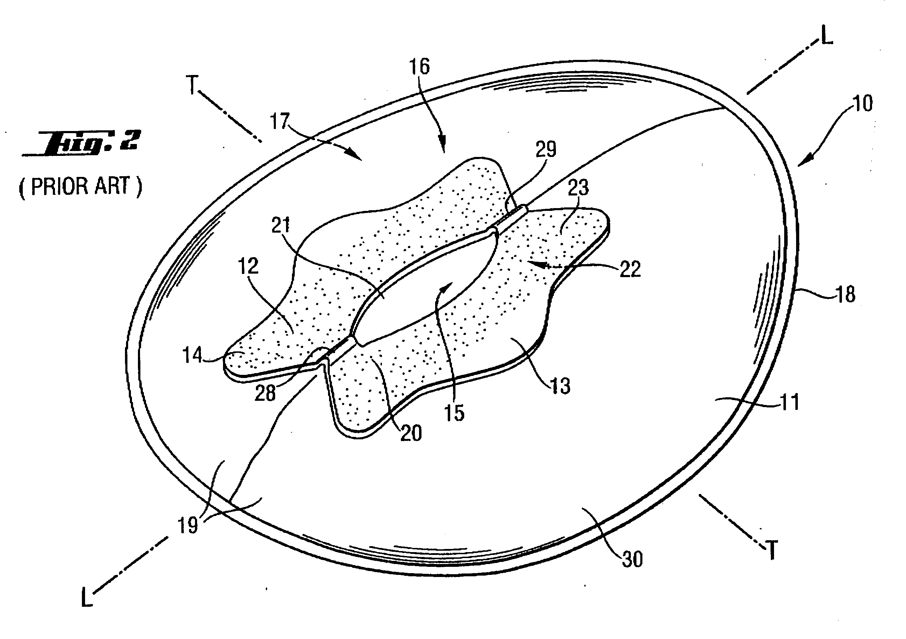

All human waste management devices according to the present invention have a flange (12) which is three dimensional in its relaxed configuration. The term three dimensional as used herein refers to a flange (12) which is not flat. Flat as used herein to describe a flange (12) which can be thought to be fully contained by a cuboid, characterised by three characterising lengths, of which one first characterising length is no more than the thickness of the material used for the flange (12). A flange (12) is also referred to as flat if only front and rear projections (28/29) can not be thought to be contained by that cuboid. Front and rear projections (28/29) are herein defined as only those projections (interchangeably to be referred to a humps) which fulfil the following condition: When seen in the longitudinal cross sectional view the end point of the hump towards the inner periphery (25) of the flange (12) has the same height on the z-axis then the end point of the hump towards the outer periphery (24) of the flange (12) as shown in Figure 2 and Figure 10 which are representative of prior art.

The present invention can be best described when considering the cross sectional views of the device (10), in particular the longitudinal cross sectional view and the transversal cross sectional view. However, points on longitudinal cross sectional views are also marked on the side views of the device (10) to make the positions of these points in relation to the device clearer.

Moreover, the present invention can be conveniently described by referring to lines of curvature, which always refer to cross sectional views and are defined as follows: Any such line comprises three points. P₁ is a point of the outer periphery of the flange (12). P₂ is a point of the inner periphery (25) of the flange (12), which is closest to P₁. Z is the point of the z axis which is closest to P₂. A line of curvature as used herein, refers to a line in a cross sectional view, which comprises the points Z, P₁ and P₂ and which is not straight. Referring for example to Figure 3, the line k₁ is a line of curvature. Similarly, k₂ , which comprises the points P₁', P₂' and Z, is a line which is not straight and thus is a line of curvature.

Furthermore, as used herein, the line k₁ depicted in Figure 3 is referred to as concave. When following k₁ from the point P₁ via point P₂ to point Z the line is either linear or bends to the left. In more mathematical terms the gradient of a function whose graph is represented by the line k₁ does not change sign along line k₁. For example, the gradient can be defined to be either negative or zero (but never positive) along line k₁. Convex, as used herein, refers to a line which has a gradient as defined above which is either positive or zero (but never negative). For example, the line m₁ in Figure 4 is referred to a convex, since m₁ is either straight or bends to the right when following m₁ from Q₁ via Q₂ to Z'.

Preferred embodiments of the present invention comprise at least one line of curvature. More preferably at least two lines of curvature, yet more preferably at least four lines of curvature are comprised. Preferably at least one, more preferably at least two lines of curvature are oriented in the longitudinal direction. Preferably at least one line of curvature oriented in the longitudinal direction is concave. Moreover preferably at least one, more preferably at least two lines of curvature are oriented in the transversal direction. Preferably at least one line of curvature oriented in the transversal direction is convex.

Figure 4 depicts a transversal cross sectional view of the above described preferred embodiment. The cross section comprises two lines of curvature: m₁, comprising Q₁, Q₂ and Z', and m₂, comprising Q₁', Q₂' and Z'. Both lines, m₁ and m₂, are convex. This reflects the contour of the human body as can be seen from line l₂ in Figure 5 and ensures the good anatomical fit of the device (10).

Another preferred embodiment of the present invention as represented by its longitudinal side view is depicted in Figure 6. The embodiment of Figure 6 is also designed to follow the contours of the human body along line l₁. This embodiment comprises two lines of curvature oriented in the longitudinal direction. Both lines of curvature are concave and are mirror images of each other.

The embodiment of Figure 6 while typically not providing an anatomical fit as good as the embodiment of Figure 3, since the line of curvature comprises only straight portions, provides the advantage of an easier and more cost effective production. To this benefit, the embodiment shown in Figure 6, comprises two pleats (34) to each side of the longitudinal axes. These pleats (34) ensure that the flange (12) which is cut from a substantially flat piece of material is provided in a three dimensional configuration comprising lines of curvature.

These pleats (34) on each side of the longitudinal axis are arranged in a rhombic pattern, so that their distance when measured along the inner periphery (25) is shorter than their distance measured along the outer periphery (24) of the flange (12).

Figure 7 shows another preferred embodiment of the present invention. The embodiment of Figure 7 comprises two elastics (26) and a skirt (27). The elastics (26) are positioned to either side of the longitudinal axis and extend in the longitudinal direction. The elastic modulus can be chosen to shorten the width of the aperture (21) as measured along the longitudinal direction. This shortening has the effect of supporting a curvature along a longitudinal line. The skirt (27) depending on its elastic modulus may further supports this.

Figure 8 shows another preferred embodiment of the present invention. The flange (12) comprises transversal lines of curvature (k₁ and k₂) which are convex. Furthermore the contour of the flange (12), when seen in the transversal cross sectional view, comprises in that order a concave portion (between P₁ and P₂), a convex portion (between P₂ and P₂') and a concave portion (between P₂' and P₁'). This design better follows the contour of the anal groove and the adjacent areas of the anus, as indicated by line l₂ of Figure 2.

Figure 9 discloses another preferred embodiment of the present invention, which comprises, e.g. as compared to Figure 3, a relatively large aperture (21) and a relatively small flange (12). This particular design has been found to perform well when used for a faecal management device (10) for a baby. The embodiment comprises to lines of curvature oriented in the longitudinal direction, which are both concave, but which are not mirror images of each other.

The person skilled in the art has no difficulty in designing a product which combines the features disclosed in different side views (from one side of the longitudinal axis) and transversal cross sectional views described herein, for instance to design a product according to Figure 3 and Figure 8 or according to Figure 9 and Figure 8.

For example the three dimensional shape of the flange (12) can be provided by providing the flange (12) with pleats (34), or by providing the flange (12) from several appropriately shaped pieces of material and/or by cutting the material for the flange (12) appropriately or by thermo moulding the flange (12) or by any other means for preforming the flange (12).

The faecal management device (10) or urine management device may be provided in combination with an applicator (40) or any other tool or aide for application, usage period and detachment. When an applicator (e.g. as described in patent application No. PCT/US98/13298,"Applicator for a faecal management device") is used for the application of a human waste management device, the applicator preferably is shaped to support the relaxed configuration, which according to the present invention is anatomically advantageous.

## Claims

1. Human waste management device comprising a flange (12) and an aperture (21), said flange (12) surrounding said aperture (21), said flange (12) having a relaxed configuration characterised in that said flange (12) in said relaxed configuration comprises at least one line of curvature as defined herein.

2. Human waste management device according to claim 1 characterised in that said line of curvature is oriented in the transversal direction.

3. Human waste management device according to claim 2, characterised in that said line of curvature is convex.

4. Human waste management device according to anyone of the preceding claims characterised in that said line of curvature is oriented in the longitudinal direction.

5. Human waste management device according to Claim 4 characterised in that said line of curvature is concave.

6. Human waste management device according to anyone of the preceding claims characterised in that said flange (12) comprises at least two lines of curvature as defined herein.

7. Human waste management device according to Claim 3, having a transversal cross sectional view characterised in that said transversal cross sectional view of said flange (12) comprises in this order a concave, a convex and a concave portion.

8. Human waste management device according to anyone of the preceding claims characterised in that said flange (12) comprises at least one pleat (34).
